# EUROPEAN PATENT APPLICATION

(11) **EP 3 167 798 A1**
(43) Date of publication of application: **17.05.2017**
(21) Application number: 15818324.4
(22) Date of filing: 10.07.2015
(51) Int. Cl.: A61B 5/04, A61B 5/0478, A61B 5/0492, A61B 5/05, A61F 2/60, A61F 2/72, G08C 19/00

(54) **BIOLOGICAL SIGNAL MEASURING DEVICE, BIOLOGICAL SIGNAL MEASURING METHOD, MOUNTED-TYPE MOTION ASSIST DEVICE, AND MOTION ASSIST METHOD**

(30) Priority: 10.07.2014 JP 2014142409
(71) Applicant: Cyberdyne Inc., Tsukuba-shi, Ibaraki 305-0818 (JP); University of Tsukuba, Tsukuba-shi, Ibaraki 305-8577 (JP)
(72) Inventor: SANKAI, Yoshiyuki, Tsukuba-shi Ibaraki 305-8577 (JP)
(74) Representative: Hoffmann Eitle
(86) International application number: PCT/JP2015/069915
(87) International publication number: WO 2016/006688

(57) **Abstract**

According to the present invention, various biological signals can be measured at an appropriate position without depending on an expert. A biological-signal measurement device includes a sensor 1 that is in contact with a nerve fiber N and detects a nerve action potential generated in the nerve fiber N, a signal processor 2 that is connected to the sensor 1 via wiring 5, the signal processor 2 obtaining a signal based on the nerve action potential from the sensor 1 and wirelessly transmitting the obtained signal, and a receiving/transmitting unit 3 that receives the signal from the signal processor 2. The sensor 1 and the signal processor 2 are embedded in a body. The receiving/transmitting unit 3 receives the signal from the signal processor 2 via skin S.

## Description

### Technical Field

The present invention relates to a biological-signal measurement device, a biological-signal measurement method, a wearable action assistance device, and an action assistance method.

### Background Art

An electrocardiogram, an electroencephalogram, an electrooculogram, an electromyogram, and the like which are obtained by measuring biopotentials, such as a cardiac potential, brain waves, an ocular potential, and a muscle potential, are important biological information for grasping the condition of a test subject or a patient (hereinafter collectively referred to as a "person subjected to measurement") in medical care. In the related art, a sensor is directly brought into contact with skin, or after gel or the like is applied on skin, a sensor is brought into contact with skin. In this manner, these biopotentials are measured. However, in this measurement method, hair in a sensor contact portion needs to be shaved, or application of gel may cause a skin rash. In measurement of biological signals, a particular (special) sensor is used. Therefore, an expert needs to perform the measurement in a hospital or another institution. Thus, in the related art, every time biological signals are measured, the measurement becomes burdensome and troublesome for a person subjected to measurement. In addition, obtained biological signals are limited due to restriction on a portion in which a sensor is installed, as in a case in which a site and the degree to which biological signals are transmitted are limited due to physiological or physical characteristics of a person subjected to measurement.

Patent Literature 1: Japanese Unexamined Patent Application Publication No. 2005-230099

### Summary of Invention

An object of the present invention is to provide a biological-signal measurement device and a biological-signal measurement method which enable various biological signals to be measured at an appropriate position without depending on an expert, and to provide a wearable action assistance device and an action assistance method which use the biological-signal measurement device.

According to one aspect of the present invention, there is provided a biological-signal measurement device including a sensor that detects a nerve action potential generated in a nerve fiber, a signal processor that is connected to the sensor via wiring, the signal processor obtaining a signal based on the nerve action potential from the sensor and wirelessly transmitting the obtained signal, and a receiving/transmitting unit that receives the signal from the signal processor.

According to one aspect of the present invention, the biological-signal measurement device further includes a stimulation sensor that provides an electrical stimulus to biological tissue. The receiving/transmitting unit transmits, to the signal processor, an instruction about a timing or strength of the electrical stimulus that is to be supplied to the biological tissue, and the signal processor generates a stimulus signal on the basis of the received instruction, and provides the electrical stimulus to the biological tissue by using the stimulation sensor.

According to one aspect of the present invention, the instruction for the electrical stimulus is determined on the basis of the nerve action potential detected by the sensor.

According to one aspect of the present invention, there is provided a biological-signal measurement method including bringing a sensor into contact with a nerve fiber and detecting a nerve action potential generated in the nerve fiber, by using a signal processor in a body, obtaining a signal based on the nerve action potential from the sensor through wiring, and by using the signal processor, wirelessly transmitting the obtained signal to a receiving/transmitting unit located outside the body.

According to one aspect of the present invention, there is provided a wearable action assistance device including a frame that is worn along a skeleton of a wearer, a driving source that supplies power to the wearer through the frame, the biological-signal measurement device according to the present invention that measures a nerve action potential generated in accordance with an instruction from a brain of the wearer, a detecting unit that detects an angle of a joint of the wearer, and a control unit that produces, in the driving source, power according to the nerve action potential and the angle of a joint.

According to one aspect of the present invention, there is provided an action assistance method using the wearable action assistance device according to the present invention. The method includes detecting, from the sensor, the nerve action potential at a position located between the brain and a torn portion of a spinal cord of the wearer, the nerve action potential being generated in accordance with the instruction from the brain, by using the signal processor, obtaining the signal based on the nerve action potential from the sensor via the wiring, by using the signal processor, wirelessly transmitting the obtained signal to the receiving/transmitting unit located outside the body, by using the receiving/transmitting unit, outputting the received signal to the control unit, and by using the control unit, producing, in the driving source, the power according to the nerve action potential and the angle of a joint.

### Advantageous Effects of Invention

The present invention enables various biological signals to be measured at an appropriate position in accordance with characteristics of a person subjected to measurement, without depending on an expert. In addition, action assistance may be provided by using measured biological signals.

### Brief Description of Drawings

[Fig. 1] Fig. 1 is a schematic diagram of a biological-signal measurement device according to a first embodiment.
[Fig. 2] Fig. 2 is a block diagram illustrating the configuration of a control system of a wearable action assistance device according to a second embodiment.
[Fig. 3] Fig. 3 is a perspective view obtained by viewing a wearer in the wearable action assistance device from the front.
[Fig. 4] Fig. 4 is a perspective view obtained by viewing the wearer in the wearable action assistance device from the back.
[Fig. 5] Fig. 5 is a diagram illustrating tasks into which actions of a wearer are classified.

### Description of Embodiments

Embodiments of the present invention will be described below on the basis of the drawings.

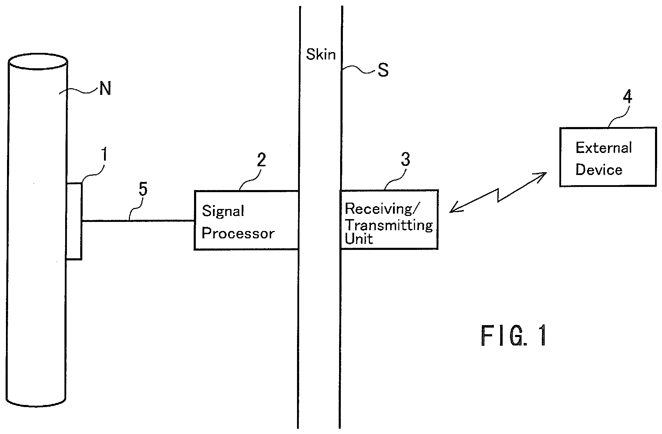

[First Embodiment] Fig. 1 is a schematic diagram of a biological-signal measurement device according to a first embodiment. The biological-signal measurement device includes a sensor 1, a signal processor 2, and a receiving/transmitting unit 3. The sensor 1 and the signal processor 2 are provided in a human body, and the receiving/transmitting unit 3 is provided outside the human body.

The sensor 1 is in contact with a nerve fiber (nerve axon) N, and detects a nerve action potential. The signal processor 2 is electrically connected to the sensor 1 via wiring (lead wire) 5, and obtains, from the sensor 1, a signal (nerve action potential) in the nerve fiber N. The signal processor 2 transmits, to the receiving/transmitting unit 3, the signal obtained from the sensor 1. The receiving/transmitting unit 3 transmits, to an external device 4, the nerve action signal received from the signal processor 2. The external device 4 utilizes the nerve action signal for diagnosis or the like.

The sensor 1 is a micro-sensor (microelectrode) including, for example, a silicon substrate and a metal material formed on the silicon substrate. As the metal material, a corrosion-proof noble metal, such as gold, platina, or the like, or an alloy of these is used. Multiple sensors 1 including a sensor detecting the ground potential and a sensor detecting a reference potential are provided.

The sensor 1 is a cylindrical or clamp sensor having a space (through hole) in which the nerve fiber N is present. A sensor unit (metal unit) is provided on the side surface of the through hole. Thus, electric contact between the sensor 1 and the nerve fiber N may be stabilized and a nerve action potential may be detected from the nerve fiber N in a stable manner. The sensor 1 may be a needle sensor, and may be stuck into the nerve fiber N.

Wiring 5 includes a conductor, such as copper or gold, and an insulating resin material covering the conductor. As the insulating resin material, a flexible and highly biocompatible material, such as silicone resin or polyurethane resin, may be used.

The signal processor 2 performs signal processing, such as noise removal and amplification, on a signal (nerve action potential) detected by the sensor 1, and wirelessly transmits the result to the receiving/transmitting unit 3. The signal processor 2 may be provided with a small driving battery, or may be provided with an antenna coil to receive, from the receiving/transmitting unit 3, driving power through wireless power transmission.

The external device 4 uses a signal received by the receiving/transmitting unit 3. For example, the external device 4 is a computer for diagnosis, and generates an electrocardiogram and an electroencephalogram. For example, the external device 4 is a robot arm, and actions of the robot arm are controlled on the basis of a muscle potential and the like detected by the sensor 1.

The sensor 1 and the signal processor 2 are embedded in a body through a surgical operation. For example, the sensor 1 is provided in such a manner as to be in contact with an axon or a dendrite in the central nervous system or the peripheral nervous system. The signal processor 2 is provided just under skin (in a hypodermic site). Near the signal processor 2, the receiving/transmitting unit 3 receives a signal from the signal processor 2 via skin S.

When a sensor is brought into contact with skin from the outside of a body, there is a restriction on where the sensor is installed. A nerve action potential fails to be efficiently detected, for example, in a deep portion of a body or inside a skull. In contrast, in the present embodiment, the sensor 1 is embedded in a body so as to be in contact with a nerve fiber. Therefore, a nerve action potential in a desired site may be efficiently detected. A signal detected by the sensor 1 is transmitted from the signal processor 2 provided under skin, to the outside of a body. Therefore, the transmitted signal may be efficiently received by the receiving/transmitting unit 3 provided outside a body.

After the sensor 1 and the signal processor 2 are embedded in a body, a detection result from the sensor 1 may be obtained in such a manner that a user merely causes the receiving/transmitting unit 3 to approach the signal processor 2. Therefore, a particular (special) operation performed by an expert is unnecessary, and a biological signal may be easily measured. The physiological condition of a user may be monitored all the time.

In the above-described embodiment, the example in which the sensor 1 detects a nerve action potential is described. The sensor 1 may provide an electrical stimulus to biological tissue. For example, the external device 4 determines a timing or the strength of an electrical stimulus that are to be provided to biological tissue, and instructs the receiving/transmitting unit 3. The receiving/transmitting unit 3 transmits the instruction supplied from the external device 4, to the signal processor 2. The signal processor 2 generates a stimulus signal on the basis of the received instruction, and causes the sensor 1 to provide an electrical stimulus to biological tissue. The biological tissue to which an electrical stimulus are provided may be a nerve cell regenerated through regenerative therapy.

The biological-signal measurement device may include the sensor 1 detecting a nerve action potential and the sensor 1 providing an electrical stimulus to biological tissue. For example, the sensor 1 for detecting a potential detects a nerve action potential in a path (for example, a spinal ascending tract) which ascends to the upper nerve center (brain). The sensor 1 for an electrical stimulus provides an electrical stimulus to a path (for example, a spinal descending tract) which descends from the upper nerve center. Thus, even when the spinal cord has been torn, an extremity site lower than the torn portion may be moved, or a sensation in the site may be obtained. An electrical stimulus that is to be provided to biological tissue may be determined on the basis of the nerve action potential detected in the ascending path, and the stimulation sensor may provide the electrical stimulus.

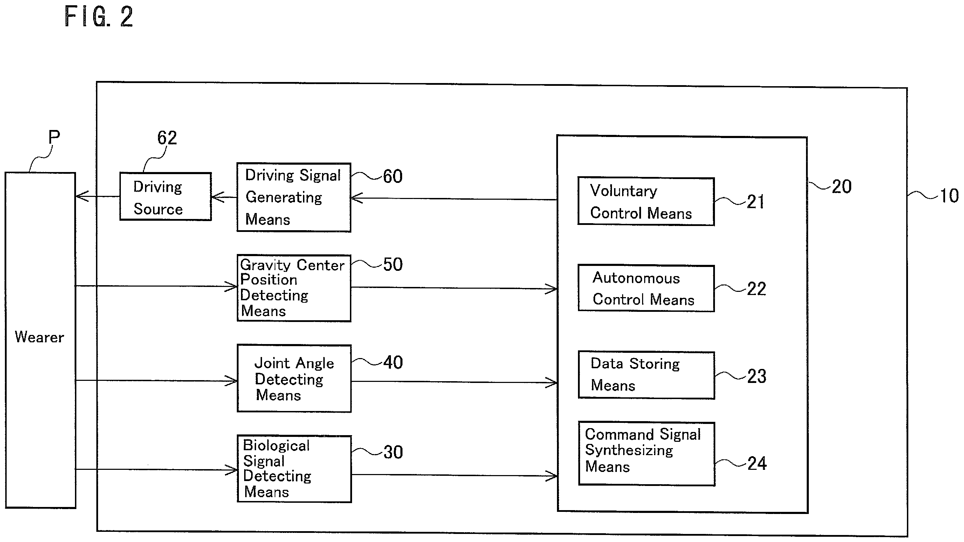

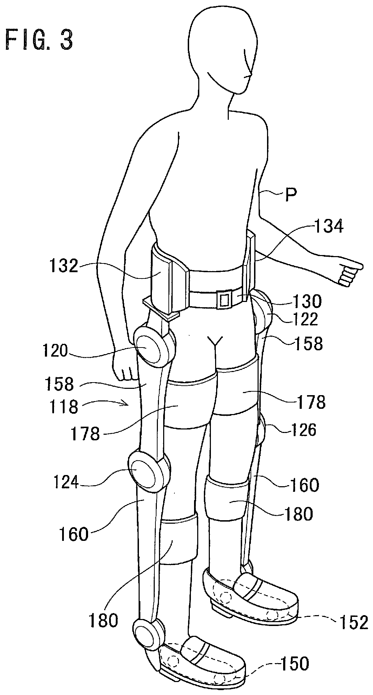

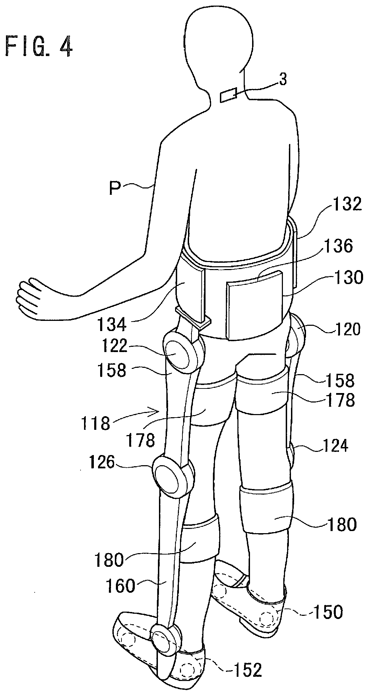

[Second Embodiment] Fig. 2 is a block diagram illustrating the configuration of a control system of a wearable action assistance device 10 according to a second embodiment. Fig. 3 is a perspective view obtained by viewing a person (wearer P) wearing the wearable action assistance device 10 from the front. Fig. 4 is a perspective view obtained by viewing the person from the back.

The wearable action assistance device 10 is obtained, for example, by connecting frames to actuator units in a rotatable manner. The frames are worn in such a manner as to extend between joints for the body of a person (wearer), such as their waist, their thighs, and their shanks. The wearable action assistance device 10 illustrated in Figs. 3 and 4 is a wearable action assistance device for a lower body which has actuator units at the knee joints and the hip joints. In the present invention, a wearable action assistance device for an upper body or the whole body may be used.

As illustrated in Fig. 2, the wearable action assistance device 10 includes a control device 20, biological-signal detecting means 30, joint angle detecting means 40, gravity center position detecting means 50, driving-signal generating means 60, and a driving source 62.

As the biological-signal detecting means 30, the biological-signal measurement device according to the first embodiment described above is used. The sensor 1 and the signal processor 2 of the biological-signal detecting means 30 are embedded in a body, and detect a biological signal (for example, a biopotential signal or brain waves) generated when the wearer P moves muscles around a joint. The receiving/transmitting unit 3 is disposed near the signal processor 2. For example, in the case where the spinal cord of the wearer P is torn, and where a nerve does not extend to a lower extremity, the sensor 1 is embedded on the upper side (the side close to the brain) higher than the torn portion, and detects a nerve action potential from a nerve fiber. In the example in Fig. 4, the receiving/transmitting unit 3 is disposed on the wearer P's neck. The receiving/transmitting unit 3 of the biological-signal detecting means 30 transmits, to the control device 20, a biological signal detected by the sensor 1.

The joint angle detecting means 40 detects the angle of a joint which is produced in an action of the wearer P, and outputs the angle to the control device 20. As the joint angle detecting means 40, an angle sensor is used. The angle sensor which includes, for example, a rotary encoder which counts pulses whose number is proportional to the angle of a joint of the wearable action assistance device 10 outputs, as a sensor output, an electric signal corresponding to the number of pulses according to the angle of a joint. Specifically, the angle sensor detects a rotation angle between two frames coupled to the driving source (actuator units) 62.

The gravity center position detecting means 50 detects the position of the center of gravity which is determined in an action of the wearer P, and outputs the result to the control device 20.

The control device 20 includes voluntary control means 21, autonomous control means 22, data storing means 23, and command signal synthesizing means 24.

The voluntary control means 21 uses a biological signal detected by the biological-signal detecting means 30, so as to generate a voluntary command signal for producing power according to the wearer P's intention in the driving source 62.

The data storing means 23 stores a reference parameter database for specifying a phase of a task performed by the wearer P, and assistance parameters for assisting an action of the wearer P in accordance with the specified phase. Tasks are obtained by classifying main action patterns of human beings. Phases are a series of minimum action units included in the tasks.

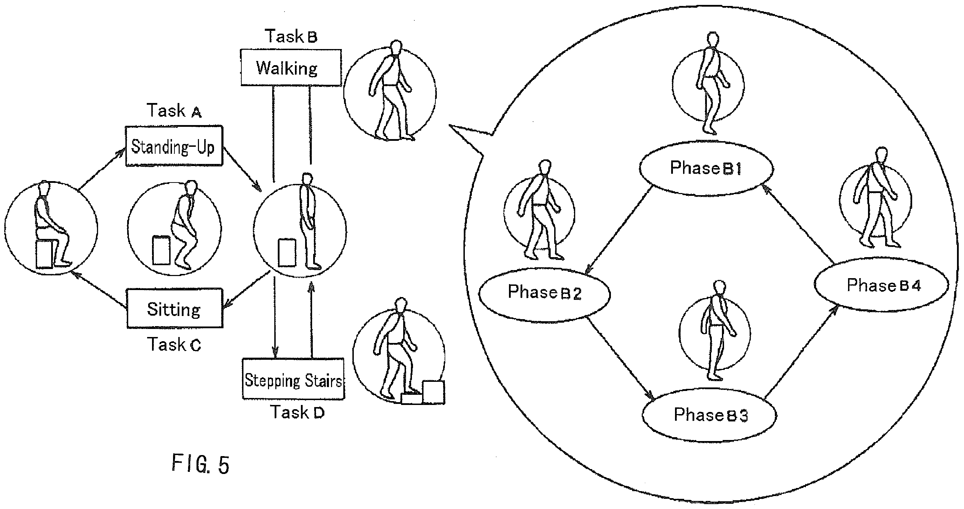

Fig. 5 illustrates exemplary tasks and phases stored in the reference parameter database.

As illustrated in Fig. 5, as tasks obtained through classification of actions of the wearer P, for example, a task A having data for a standing-up action in which a shift from a sitting state to a standing state is produced, a task B having data for a walking action in which the wearer P who has stood up walks, a task C having data for a sitting action in which a shift from a standing state to a sitting state is produced, and a task D having data for a stepping-stairs action in which a shift from a standing state to a stepping-stairs state is produced are stored in the reference parameter database.

Each task is provided with multiple pieces of phase data. For example, phases B1 to B4 are set in the task B for a walking action. The phase B1 has data (such as the angle of a joint and a track of the position of the center of gravity, a change in torque, and a change in a biopotential signal) for an action performed when, in a state in which a person stands with the center of gravity being on their left leg, their right leg is going forward. The phase B2 has data for an action performed when their right leg which has gone forward touches the ground and the center of gravity is shifted to their right leg. The phase B3 has data for an action performed when, in a state in which the person stands with the center of gravity being on their right leg, their left leg is going forward. The phase B4 has data for an action performed when their left leg which has gone forward of their right leg touches the ground and the center of gravity is shifted to their left leg.

Thus, analysis of typical actions of a person reveals that a typical action pattern, such as the angle of each joint and a shift of the center of gravity, has been determined in each phase. Accordingly, for each of the phases included in a large number of basic actions (tasks) of a person, a typical change of the angle of a joint, a typical condition of a shift of the center of gravity, and the like are empirically obtained, and are stored in the reference parameter database.

Multiple assistance patterns are assigned to each phase, and the assistance patterns provide assistance operations different from each other even in the same phase.

For example, a person has different walking patterns depending on the size of the body and the condition of muscle force, or depending on a walking speed or the like. In addition, the person also has different walking patterns depending on a purpose of an action (such as for rehabilitation, for training, for improvement of a gait, or for assisting an action (in terms of force)). Even for rehabilitation, suitable action patterns are different depending on the degree of a disability of a target person or progress of rehabilitation.

Therefore, different assistance operations suitable for a target action pattern are provided. Thus, a large number of assistance patterns are assigned to each phase so that an optimal assistance pattern may be selected from the multiple assistance patterns in accordance with assistance suitable for the target action pattern.

The autonomous control means 22 compares a reference parameter stored in the data storing means 23 with a parameter representing the condition of an action of the wearer P, such as the angle of a joint which is detected by the joint angle detecting means 40 and the position of the center of gravity which is detected by the gravity center position detecting means 50. Then, the autonomous control means 22 specifies the task and phase of the action of the wearer P. After the autonomous control means 22 specifies the phase in accordance with the condition of the action of the wearer P, the autonomous control means 22 selects an optimal assistance pattern from the assistance patterns assigned to the phase, in accordance with the predetermined purpose, and generates an autonomous command signal for producing power according to the assistance pattern in the driving source 62.

The command signal synthesizing means 24 synthesizes the voluntary command signal generated by the voluntary control means 21 with the autonomous command signal generated by the autonomous control means 22, and outputs the synthesized command signal to the driving-signal generating means 60. A ratio of synthesis of a voluntary command signal with an autonomous command signal may be predetermined for each phase of the tasks, and may be stored in the data storing means 23.

A synthesized command signal has a waveform for producing, in the driving source 62, power obtained by synthesizing power which is produced through voluntary control and which changes from the start to the end of an action with power obtained for each phase through autonomous control.

The driving-signal generating means 60 generates a driving signal (driving current) according to the synthesized command signal, and supplies the resulting signal to the driving source 62, thereby driving the driving source 62. The driving source 62 provides, to the wearer P, assistance force (power) according to the driving signal.

The wearable action assistance device 10 illustrated in Figs. 3 and 4 is a device which helps in performing (assists) a walking action of a person who has difficulty in walking by themselves, such as a person who has lower-extremity disabilities and who has difficulty in walking, for example, due to a torn spinal cord. In order to generate muscle force, the wearable action assistance device 10 detects a signal (biological signal) which is output from their brain, at a position higher than the torn portion of their spinal cord, and operates so that driving power from the actuators is supplied to the wearer P on the basis of the detected biological signal.

When the wearer P wearing the wearable action assistance device 10 is voluntarily going to perform a walking action, the action assistance device 10 supplies the wearer P with a driving torque which is necessary for the walking action and which serves as assistance force, in accordance with a biological signal generated at that time. Therefore, even when a nerve does not extend to a lower extremity, the actuators supply the wearer P with driving torque in accordance with a biological signal based on their intention, and the wearer P is capable of walking by moving their legs.

At that time, the action assistance device 10 exerts control so that assistance force supplied in accordance with a shift of the center of gravity which is produced in a walking action reflects the wearer P's intention. Therefore, the actuators of the action assistance device 10 are controlled so as not to supply load which acts against the wearer P's intention, and an action of the wearer P is not hindered.

As illustrated in Figs. 3 and 4, the wearable action assistance device 10 is obtained by providing driving units for a frame mechanism 118 worn by the wearer P. The driving units include a right-thigh driving power unit 120 located at the right hip joint of the wearer P, a left-thigh driving power unit 122 located at the left hip joint, a right-knee driving power unit 124 located at the right knee joint, and a left-knee driving power unit 126 located at the left knee joint. Each of these driving power units 120, 122, 124, and 126 includes an electric power unit having a DC motor, an AC motor, or the like which controls a driving torque by using a control signal from the control device, and, although being small, may supply enough driving power. Needless to say, as a driving power unit, an ultrasonic motor which is slimmed down so that the installation space is small may be used. Figs. 3 and 4 illustrate the example in which four power units are provided as driving units. As long as at least one of the power units is provided, any configuration may be employed.

Rotating shafts of the driving power units 120, 122, 124, and 126 are configured so as to transmit driving torque to first frames 158 and second frames 160 which are driven through gear mechanisms. The first frames 158 are formed in such a manner as to extend along the outside of the thighs of the wearer P. The second frames 160 are formed in such a manner as to extend downward of the first frames 158 and along the outside of the shanks of the wearer P.

Thigh fastening members 178 which form as belts and which are fastened to the thighs of the wearer P are mounted at an intermediate position in the longitudinal direction of the first frames 158. Shank fastening members 180 which form as belts and which are fastened to the shanks under the knees of the wearer P are mounted at an intermediate position in the longitudinal direction of the second frames 160. Accordingly, driving torque generated by the driving power units 120, 122, 124, and 126 is transmitted through gears to the first frames 158 and the second frames 160, and is further transmitted as assistance force through the thigh fastening members 178 and the shank fastening members 180 to the legs of the wearer P.

On a waist fastening member 130 which forms as a belt and which is worn on the waist of the wearer P, batteries 132 and 134 which serve as power supplies for driving the driving power units 120, 122, 124, and 126 are mounted. The batteries 132 and 134 are rechargeable batteries, and are disposed in a distributed manner on the left and right so as not to hinder an action of the wearer P.

On the rear side of the waist fastening member 130 which is on the backside of the wearer P, a control unit 136 is mounted. The control unit 136 houses the control device 20 described above.

The biological-signal detecting means 30 includes the sensor 1, the signal processor 2, and the receiving/transmitting unit 3. From a nerve fiber at a position higher than the torn portion of the spinal cord, the sensor 1 detects a biological signal (nerve action potential) transmitted to the legs of the wearer P from the brain in order to move the legs. The signal processor 2 obtains the biological signal from the sensor 1 for transmission. The receiving/transmitting unit 3 receives the biological signal from the signal processor 2. The sensor 1 and the signal processor 2 are embedded in the body of the wearer P. Therefore, the sensor 1 and the signal processor 2 are not illustrated in Figs. 3 and 4. The receiving/transmitting unit 3 is disposed near the signal processor 2, for example, on the backside of the neck. The receiving/transmitting unit 3 transmits a detected biological signal to the control device 20 in the control unit 136. The receiving/transmitting unit 3 may be housed in the control unit 136. Detection of an action potential generated in accordance with an instruction from the brain enables estimation of muscle force that is to be generated in an action, and enables assistance force necessary for an action to be obtained from a virtual torque based on the estimated muscle force.

Therefore, the control device 20 is configured to obtain driving current supplied to the four driving power units 120, 122, 124, and 126 on the basis of a biological signal detected by the biological-signal measurement device serving as the biological-signal detecting means 30, and supply assistance force and assist an action of the wearer P by driving the driving power units 120, 122, 124, and 126 by using the driving current.

Each of the driving power units 120, 122, 124, and 126 has the joint angle detecting means 40 for detecting the angle of a joint. The joint angle detecting unit 40 which includes an angle sensor such as a rotary encoder which obtains the number of pulses which is proportional to the angle of a joint outputs, as a sensor output, an electric signal corresponding to the number of pulses according to the angle of a join.

To shift the center of gravity smoothly, it is necessary to detect load borne on the back surfaces of the feet and detect the position of the center or gravity. Therefore, on the back surfaces of the left and right feet of the wearer P, load measuring units 150 and 152 (illustrated by using dashed lines in Figs. 3 and 4) which measure load at least two points on the back surfaces of the feet are provided. The load measuring units 150 and 152 are configured to be held so as to be in contact with the back surfaces of the feet, and to detect reaction force which changes in accordance with weight shift produced in a walking action.

Thus, according to the present embodiment, even in the case where the spinal cord of the wearer P is torn and where a nerve does not extend to a lower extremity, the sensor 1 of the biological-signal measurement device is embedded at a position higher than the torn portion, and a biological signal (nerve action potential) based on an instruction from the brain is detected. Thus, the wearable action assistance device 10 may help in performing (assist) a walking action.

In the above-described first or second embodiment, when a person subjected to measurement moves their body member or reacts to an external stimulus, the biological-signal measurement device or the biological-signal detecting means 30 may detect a biological signal (such as a biopotential signal) that is fed back from the peripheral nervous system to the central nervous system. A biological signal or the like which passes through the central nervous system including the brain and through a nerve circuit which is connected through the spinal cord and a motor nerve to the peripheral nervous system may be measured in both of the direction from the central nervous system to the peripheral nervous system and the direction from the peripheral nervous system to the central nervous system.

This makes it possible to check if a signal transmitted from the brain is received by the peripheral nervous system without failure and if information from the peripheral nervous system is fed back to the brain without failure.

The present invention is described in detail by using specific embodiments. It should be clear for a person skilled in the art that various changes may be made without departing from the intention and scope of the present invention.

The present application is based on Japanese Patent Application No. 2014-142409 filed on July 10, 2014, the contents of which are incorporated herein by reference in their entirety.

### Reference Signs List

- 1: sensor
- 2: signal processor
- 3: receiving/transmitting unit
- 4: external device

## Claims

1. A biological-signal measurement device comprising:
a sensor that detects a nerve action potential generated in a nerve fiber;
a signal processor that is connected to the sensor via wiring, the signal processor obtaining a signal based on the nerve action potential from the sensor and wirelessly transmitting the obtained signal; and
a receiving/transmitting unit that receives the signal from the signal processor.

2. The biological-signal measurement device according to Claim 1, further comprising:
a stimulation sensor that provides an electrical stimulus to biological tissue,
wherein the receiving/transmitting unit transmits, to the signal processor, an instruction about a timing or strength of the electrical stimulus that is to be supplied to the biological tissue, and
wherein the signal processor generates a stimulus signal on the basis of the received instruction, and provides the electrical stimulus to the biological tissue by using the stimulation sensor.

3. The biological-signal measurement device according to Claim 2, wherein the instruction for the electrical stimulus is determined on the basis of the nerve action potential detected by the sensor.

4. A biological-signal measurement method comprising:
bringing a sensor into contact with a nerve fiber and detecting a nerve action potential generated in the nerve fiber;
by using a signal processor in a body, obtaining a signal based on the nerve action potential from the sensor through wiring; and
by using the signal processor, wirelessly transmitting the obtained signal to a receiving/transmitting unit located outside the body.

5. A wearable action assistance device comprising:
a frame that is worn along a skeleton of a wearer;
a driving source that supplies power to the wearer through the frame;
the biological-signal measurement device according to Claim 1 that measures a nerve action potential generated in accordance with an instruction from a brain of the wearer;
a detecting unit that detects an angle of a joint of the wearer; and
a control unit that produces, in the driving source, power according to the nerve action potential and the angle of a joint.

6. An action assistance method using the wearable action assistance device according to Claim 5, the method comprising:
detecting, from the sensor, the nerve action potential at a position located between the brain and a torn portion of a spinal cord of the wearer, the nerve action potential being generated in accordance with the instruction from the brain;
by using the signal processor, obtaining the signal based on the nerve action potential from the sensor via the wiring;
by using the signal processor, wirelessly transmitting the obtained signal to the receiving/transmitting unit located outside the body;
by using the receiving/transmitting unit, outputting the received signal to the control unit; and
by using the control unit, producing, in the driving source, the power according to the nerve action potential and the angle of a joint.
